# EUROPÄISCHE PATENTSCHRIFT

(11) **EP 0 391 205 B1**
(45) Veröffentlichungstag und Bekanntmachung des Hinweises auf die Patenterteilung: **25.05.1994**
(21) Anmeldenummer: 90105753.9
(22) Anmeldetag: 27.03.1990
(51) Int. Cl.: C07D 213/61

(54) **Verfahren zur Herstellung von 2-Chlor-5-aminomethylpyridin**
Process for the preparation of 2-chloro-5-aminomethyl-pyridine
Procédé de préparation de la 2-chloro-5-aminométhylpyridine

(30) Priorität: 07.04.1989 DE 3911224
(43) Veröffentlichungstag der Anmeldung: 10.10.1990
(73) Patentinhaber: BAYER AG, 51368 Leverkusen (DE)
(72) Erfinder: Diehr, Hans-Joachim, Dr., D-5600 Wuppertal 11 (DE)

(56) Entgegenhaltungen:
- EP-A- 0 302 389
- DE-A- 3 726 993
- DE-A- 3 727 126
- US-A- 4 499 097

## Beschreibung

Die Erfindung betrifft ein neues Verfahren Zur Herstellung des bekannten 2-Chlor-5-aminomethyl-pyridins.

Es ist bekannt, daß man 2-Chlor-5-aminomethyl-pyridin durch Umsetzung von 2-Chlor-5-cyanopyridin in Gegenwart von Raney-Nickel und Ammoniak mit Wasserstoff erhält (vgl. DE-OS 3 727 126).

Es ist bekannt, daß man 2-Chlor-5-aminomethyl-pyridin, ein Zwischenprodukt für die Herstellung von Insektiziden, erhält, wenn man in einer ersten Stufe 2-Chlor-5-chlormethyl-pyridin mit Phthalimid in Gegenwart von Kaliumhydroxid, Dimethylformamid und Ethanol zu N-(2-Chlor-pyridin-5-yl-methyl)-phthalimid umsetzt und daraus in einer zweiten Stufe mit Hydrazinhydrat in Ethanol das gewünschte 2-Chlor-5-aminomethyl-pyridin freisetzt (vgl. EP-A 302389, S. 23 und DE-OS 3 727 126).

Weiterhin ist 2-Chlor-5-aminomethyl-pyridin als Zwischenprodukt zur Herstellung von Hypotensiva bekannt (vgl. US-P. 4 499 097).

Es bestand nun ein bislang noch nicht befriedigter Bedarf nach einem einfacheren, möglichst einstufigen Herstellungsverfahren für 2-Chlor-5-aminomethyl-pyridin ausgehend von 2-Chlor-5-chlormethyl-pyridin.

Es wurde ein Verfahren zur Herstellung von 2-Chlor-5-aminomethyl-pyridin der Formel (I) 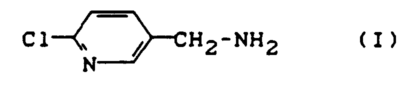
gefunden, welches dadurch gekennzeichnet ist, daß man 2-Chlor-5-chlormethyl-pyridin der Formel (II) 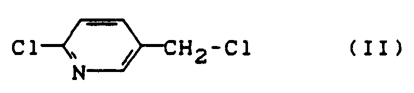
mit überschüssigem Ammoniak, gegebenenfalls in Gegenwart eines Verdünnungsmittels, bei Temperaturen zwischen -50°C und +50°C umsetzt.

Es ist als überraschend anzusehen, daß das erfindungsgemäße Verfahren in hoher Selektivität zum Produkt der Formel (I) führt, da auch mit einer nucleophilen Verdrängung des zweiten Chlor-Substituenten zu rechnen war.

Vorteile des neuen Verfahrens liegen in seiner einfachen Durchführbarkeit sowie in der guten Ausbeute und hohen Qualität des Produktes.

Der Reaktionsablauf beim erfindungsgemäßen Verfahren kann durch das folgende Formelschema wiedergegeben werden: 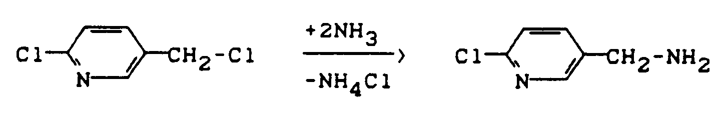
Das beim erfindungsgemäßen Verfahren als Ausgangsstoff Zu verwendende 2-Chlor-5-chlormethyl-pyridin der Formel (II) ist bereits bekannt (vgl. US-P 4332944; J. Heterocycl. Chem, 16 (1979), 333-337).

Das erfindungsgemäße Verfahren wird vorzugsweise unter Verwendung eines Verdünnungsmittels durchgeführt. Als Verdünnungsmittel kommen dabei praktisch alle inerten organischen Losungsmittel in Frage. Hierzu gehören vorzugsweise aliphatische und aromatische, gegebenenfalls halogenierte Kohlenwasserstoffe, wie Hexan, Heptan, Octan, Cyclohexan, Methylcyclohexan, Benzol, Toluol, Xylol, Chlorbenzol und o-Dichlorbenzol, Ether wie Diethylether, Dipropylether, Diisopropylether, Dibutylether, Diisobutylether, Tetrahydrofuran, Dioxan, Glykoldimethylether und Diglykoldimethylether.

Toluol wird als Verdünnungsmittel ganz besonders bevorzugt.

Die Reaktionstemperaturen können bei dem erfindungsgemäßen Verfahren in einem größeren Bereich variiert werden. Im allgemeinen arbeitet man bei Temperaturen zwischen -50°C und +50°C, vorzugsweise bei Temperaturen zwischen -35°C und +25°C.

Das erfindungsgemäße Verfahren wird im allgemeinen bei Normaldruck oder unter erhöhtem Druck bis etwa 20 bar, vorzugsweise bis zu 10 bar, durchgeführt.

Zur Durchführung des erfindungsgemäßen Verfahrens setzt man auf 1 Mol 2-Chlor-5-chlormethyl-pyridin der Formel (II) im allgemeinen zwischen 100 ml und 2000 ml, vorzugsweise zwischen 300 ml und 1500 ml Ammoniak (flüssig) ein.

Zur Durchführung des erfindungsgemäßen Verfahrens können das 2-Chlor-5-chlormethyl-pyridin, das Ammoniak und das Verdünnungsmittel in beliebiger Reihenfolge zusammengegeben werden.

In einer bevorzugten Ausführungsform des erfindungsgemäßen Verfahrens wird das Ammoniak (flüssig) in einem Autoklaven vorgelegt und das 2-Chlor-5-chlormethyl-pyridin wird, vorzugsweise in einem geeigneten Verdünnungsmittel gelöst oder suspendiert, langsam zudosiert. Das Reaktionsgemisch wird bis zum Ende der Umsetzung gerührt und dann nach üblichen Methoden aufgearbeitet (vgl. Herstellungsbeispiel).

### Herstellungsbeispiel:

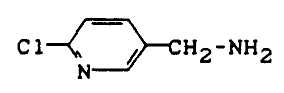

1 l Ammoniak (flüssig) wird in einem Autoklaven vorgelegt und eine auf 0°C bis 5°C abgekühlte Lösung von 176 g (92%ig, 1 Mol) 2-Chlor-5-chlormethyl-pyridin in 0,5 l Toluol innerhalb von etwa 20 Minuten mit einer Pumpe eindosiert (Druck im Autoklaven maximal etwa 10 bar). Das Reaktionsgemisch wird 8 Stunden bei 0°C bis 5°C gerührt und nach Entspannung zu einem Gemisch aus 250 ml 45%iger wässriger Natronlauge und 500 ml Toluol gegeben.

Die organische Phase wird nach Durchschütteln abgetrennt, die wässrige Phase noch einmal mit Toluol geschüttelt und die organischen Phasen werden dann vereinigt. Nach Einengen im Wasserstrahlvakuum wird der verbleibende Rückstand (146 g) im Vakuum bei 3-4 mbar destilliert.

Man erhält 102 g (Gehalt nach GC 97,3%, Ausbeute: 70% der Theorie) 2-Chlor-5-aminomethyl-pyridin vom Siedebereich 112°C-114°C (3-4 mbar).

## Patentansprüche

1. Verfahren zur Herstellung von 2-Chlor-5-aminomethyl-pyridin der Formel (I) 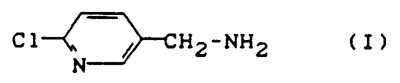 dadurch gekennzeichnet, daß man 2-Chlor-5-chlormethyl-pyridin der Formel (II) 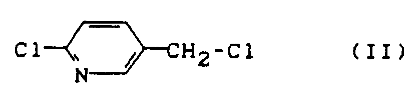 mit überschüssigem Ammoniak, gegebenenfalls in Gegenwart eines Verdünnungsmittels, bei Temperaturen zwischen -50°C und +50°C umsetzt.

2. Verfahren gemäß Anspruch 1, dadurch gekennzeichnet, daß es in Gegenwart eines inerten organischen Lösungsmittels durchgeführt wird.

3. Verfahren gemäß Anspruch 1 bis 2, dadurch gekennzeichnet, daß es in Gegenwart von Toluol durchgeführt wird.

4. Verfahren gemäß Anspruch 1 bis 3, dadurch gekennzeichnet, daß es bei Temperaturen zwischen -35°C und +25°C durchgeführt wird.

5. Verfahren gemäß Anspruch 1 bis 4, dadurch gekennzeichnet, daß es unter Normaldruck durchgeführt wird.

6. Verfahren gemäß Anspruch 1 bis 4, dadurch gekennzeichnet, daß es unter erhöhtem Druck bis etwa 20 bar durchgeführt wird.

7. Verfahren gemäß Anspruch 1 bis 6, dadurch gekennzeichnet, daß man auf 1 Mol 2-Chlor-5-chlormethy-pyridin zwischen 100 und 2000 ml flüssiges Ammoniak einsetzt.

8. Verfahren gemäß Anspruch 1 bis 6, dadurch gekennzeichnet, daß man auf 1 Mol 2-Chlor-5-chlormethyl-pyridin zwischen 300 und 1500 ml flüssiges Ammoniak einsetzt.

## Claims

1. Process for the preparation of 2-chloro-5-amino-methyl-pyridine of the formula (I) 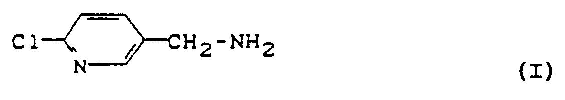 characterized in that 2-chloro-5-chloromethyl-pyridine of the formula (II) 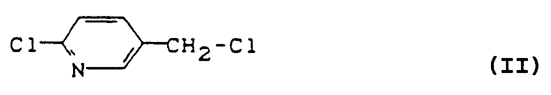 is reacted with excess ammonia, if appropriate in the presence of a diluent, at temperatures between -50°C and +50°C.

2. Process according to Claim 1, characterized in that it is carried out in the presence of an inert organic solvent.

3. Process according to Claims 1 and 2, characterized in that it is carried out in the presence of toluene.

4. Process according to Claims 1 to 3, characterized in that it is carried out at temperatures between -35°C and +25°C.

5. Process according to Claims 1 to 4, characterized in that it is carried out under normal pressure.

6. Process according to Claims 1 to 4, characterized in that it is carried out under an elevated pressure of up to 20 bar.

7. Process according to Claims 1 to 6, characterized in that between 100 and 2000 ml of liquid ammonia are employed per mole of 2-chloro-5-chloromethyl-pyridine.

8. Process according to Claims 1 to 6, characterized in that between 300 and 1500 ml of liquid ammonia are employed per mole of 2-chloro-5-chloromethyl-pyridine.

## Revendications

1. Procédé de production de 2-chloro-5-amino-méthyl-pyridine de formule (I) 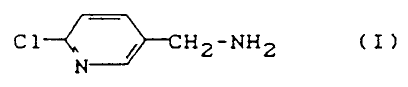 caractérisé en ce qu'on fait réagir à des températures comprises entre -50°C et +50°C de la 2-chloro-5-chlorométhyl-pyridine de formule (II) 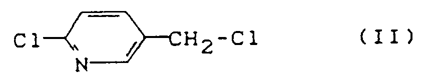 avec de l'ammoniac en excès, le cas échéant en présence d'un diluant.

2. Procédé suivant la revendication 1, caractérisé en ce qu'il est mis en oeuvre en présence d'un solvant organique inerte.

3. Procédé suivant les revendications 1 et 2, caractérisé en ce qu'il est mis en oeuvre en présence de toluène.

4. Procédé suivant les revendications 1 à 3, caractérisé en ce qu'il est mis en oeuvre à des températures comprises entre -35°C et +25°C.

5. Procédé suivant les revendications 1 à 4, caractérisé en ce qu'il est mis en oeuvre sous pression normale.

6. Procédé suivant les revendications 1 à 4, caractérisé en ce qu'il est mis en oeuvre sous pression élevée jusqu'à 20 bars.

7. Procédé suivant les revendications 1 à 6, caractérisé en ce qu'on utilise par mole de 2-chloro-5-chlorométhyl-pyridine une quantité de 100 à 2000 ml d'ammoniac liquide.

8. Procédé suivant les revendications 1 à 6, caractérisé en ce qu'on utilise par mole de 2-chloro-5-chlorométhyl-pyridine entre 300 et 1500 ml d'ammoniac liquide.
